(19)

Europäisches Patentamt

European Patent Office

Office européen des brevets

(11) **EP 3 444 610 A2**

(12) **EUROPEAN PATENT APPLICATION**

(43) Date of publication:
**20.02.2019 Bulletin 2019/08**

(51) Int Cl.:
***G01N 33/14*** *(2006.01)*

(21) Application number: **18189474.2**

(22) Date of filing: **17.08.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.08.2017 CZ 20170476**

(71) Applicant: **Vyzkumny ustav pivovarsky a sladarsky, a.s.**
**120 00 Praha 2, Nove Mesto (CZ)**

(72) Inventors:
- **Olsovska, Jana**
  **150 00 Praha 5, Radlice (CZ)**
- **Vrzal, Tomas**
  **250 01 Brandys nad Labem-Stara Boleslav (CZ)**
- **Sterba, Karel**
  **142 00 Praha 4, Kamyk (CZ)**
- **Jurkova, Marie**
  **130 00 Praha 3, Vinohrady (CZ)**

(74) Representative: **Musil, Dobroslav**
**Zabrdovicka 801/11**
**615 00 Brno (CZ)**

(54) **METHOD FOR IN SITU OBJECTIVE DETERMINATION OF THE INTENSITY OF THE OLD TASTE OF BEER**

(57) The invention relates to a method for *in situ* objective determination of the intensity of the old taste of beer, in which by means of reflectometric analysis which takes place for a period of 3 to 5 minutes, is determined the apparent sum of furan-2-carbaldehydes, in particular 5-(hydroxymethyl)furan-2-carbaldehyde and furan-2-carbaldehyde in a beer sample, and a control sample consisting of an aqueous solution of 5-(hydroxymethyl)furan-2-carbaldehyde at a concentration of 1 to 60 mg/l, and during subsequent processing of the reflectometric response thus obtained by a specific algorithm or algorithms, the stale flavor intensity of beer is quantified on the numerical scale 0-4, in exceptional cases it cannot be excluded that the value is higher than 4. The method quantification limit is 1.0, its uncertainty is 0.5.

Fig. 1

**EP 3 444 610 A2**

**Description**

**Technical field**

The invention relates to a method for *in situ* objective determination of the intensity of the old taste of beer.

**Background art**

Sensory deterioration of beer is a relatively complex phenomenon, whose essential part is, among other things, the development of the "stale flavour" of beer which consists in reducing the bitterness of beer, changing its character and at the same time in the development and growth of undesired flavours and aromas - sweet, caramel, honey, sherry, paper, cardboard, etc. The development of stale flavour is accelerated by poor beer storage, especially at temperatures higher than recommended by the manufacturer. However, if the storage temperature is, for example, 10 ° C higher than recommended, the aging process of beer is accelerated 2 to 3 times (see Arrhenius law). In addition to storage temperature and storage duration, an important role in the beer aging is played by the chemical composition of beer which is given by the composition of the input raw materials and the manufacturing technology used.

The basis of beer aging is the so-called "Maillard reaction", in which pentose and hexose present in beer react with amino groups to form in a gradual manner furan-2-carbaldehydes, especially furan-2-carbaldehyde and 5-(hydroxymethyl)furan-2-carbaldehyde. Nowadays, concentrations of these substances in beer can be monitored and, if necessary, controlled by known separation methods, for example, by liquid chromatography (HPLC) with spectrometric detection in UV spectrum. However, these methods require well-equipped laboratories, experienced professional staff and a long time for sample preparation and analysis. For these reasons, they are not applicable to *in situ* inspections.

In addition to separation methods, the extent of sensory deterioration of beer can also be judged by a panel of expert evaluators on the basis of the development of characteristic aromas. However, to obtain data validity, it is necessary to have a team of at least 7 trained experts, preferably 12, which makes also this procedure completely unusable for *in situ* inspection.

Currently, there is no objective method for determining the intensity of the old taste of beer and indirectly also its sensory deterioration which would be applicable directly on the spot, e.g. in a beer storage facility, in a brewery, in a pub or restaurant, etc. The aim of the invention is therefore to propose such a method.

**Principle of the invention**

The method for *in situ* objective determination of the intensity of beer stale flavour according to the invention is based on the detection and quantification of the apparent sum of furan-2-carbaldehydes, in particular 5-(hydroxymethyl)furan-2-carbaldehyde and furan-2-carbaldehyde, in a sample of beer by means of reflectometric analysis, and the subsequent processing of the reflectometric response thus obtained by a specific algorithm or algorithms (see below). The outcome of this method is objective numerical quantification of the intensity of beer stale flavour, which at the same time expresses an extent of sensory deterioration of beer. By adjusting the regression coefficients of the algorithm/algorithms used, the intensity of the stale flavour, or, in other words, sensory deterioration of beer can be quantified on any numerical scale, but for the purposes of explaining the invention, a scale of 0-4 has been selected; in exceptional cases, however, reaching values higher than 4 cannot be excluded. The method quantification limit is 1.0, the uncertainty is 0.5.

The method according to the invention leads to a quick outcome, is easy to use and evaluate, and when using a mobile reflector, it can be performed *in situ* - e.g. in a beer storage, brewery, restaurant, etc., and its results are fully comparable with the separation methods used so far as well as with the expert panel method (see examples 1 to 3 below). At the same time, this method is applicable to all types of top and bottom fermented beers, including low-alcohol and non-alcoholic beers, excluding dark beers.

In the method for *in situ* objective determination of the intensity of the stale flavour of beer according to the invention, the apparent sum, or, more specifically, the apparent concentration of furan-2-carbaldehydes, especially 5-(hydroxymethyl)furan-2-carbaldehyde and furan-2-carbaldehyde in a beer sample and in a control sample of an aqueous solution of 1-(hydroxymethyl) furan-2-carbaldehyde mg/l is determined by reflectometric analysis. This concentration of 5-(hydroxymethyl)furan-2-carbaldehyde in the control sample corresponds to a normal linear range of a standard reflectometer and, at the same time, corresponds to the fact that a value of the sum of furan-2-carbaldehyde in the beer higher than 60 mg/l has not been experimentally found yet. However, it is preferable if the concentration of 5-(hydroxymethyl)furan-2-carbaldehyde in the control sample is as close as possible to the presumed apparent sum of furan-2-carbaldehydes in a beer sample, since in this case the correction of the reflectometric measurement is specified with the aid of this control sample. The concentration of 5-(hydroxymethyl)furan-2-carbaldehyde in a control sample is preferably 1 to 20 mg/l, more preferably 1.5 to 7 mg/l.

The reflectometric analysis takes place for 3 to 5 minutes; comparable results are achieved within this interval. Preferably, it takes about 3 minutes, because longer analysis time involves a certain risk of a gradual decline in the reflectometric signal and distortion of the results. In addition, shorter analysis time allows to perform multiple analyses per unit of time.

If the thus detected reflectometric response of 5-(hydroxymethyl)furan-2-carbaldehyde in a control sample is equal to its actual concentration known in advance, the stale flavour intensity of beer is determined according to the formula:

$$\text{stale flavour intensity} = \alpha \cdot \ln(\ln(R)) + \beta$$

where R is the apparent sum of furan-2-carbaldehydes in the beer sample detected by reflectometric analysis, expressed in mg/l, and $\alpha$ and $\beta$ are regression coefficients, whereby $\alpha$ ranges from 0.6 to 14.2 and $\beta$ ranges from -6.5 to 2.2 depending on the type of beer being tested and on the manufacturing technology. In this case the stale flavour intensity of beer is in the range from 0 to 4, exceptionally even over 4, wherein 0 denotes undamaged, fresh beer without a trace of stale flavour, 1 corresponds to beer with a very mild stale flavour, 2 to beer with a mean stale flavour, 3 to beer with a strong stale flavour and 4 or more denotes beer with a very strong stale flavour.

If the thus determined reflectometric response of 5-(hydroxymethyl)furan-2-carbaldehyde in the control sample is not equal to its actual concentration known in advance, first the apparent sum of furan-2-carbaldehydes detected by the reflectometric analysis in the beer sample is corrected according to the formula:

$$R_{cor.} = R_{sample} - (R_{QC} - conc._{QC}),$$

wherein $Rc_{or.}$ is the corrected apparent sum of furan-2-carbaldehydes in the control sample expressed in mg/l, $R_{sample}$ is the apparent sum of furan-2-carbaldehydes in the beer sample detected by reflectometric analysis expressed in mg/l, $R_{QC}$ is the reflectometric response of 5-(hydroxymethyl)furan-2-carbaldehyde in the control sample detected by reflectometric analysis expressed in mg/l and conc.$_{QC}$ is the actual known concentration of 5-(hydroxymethyl)furan-2-carbaldehyde in the control sample expressed in v mg/l.

The stale flavour intensity is subsequently determined according to the formula:

$$\text{stale flavour intensity} = \alpha \cdot \ln(\ln(R_{cor})) + \beta$$

wherein $R_{cor.}$ is the corrected apparent concentration of furan-2-carbaldehydes in a beer sample expressed in mg/l, and $\alpha$ and $\beta$ j are regression coefficients, whereby $\alpha$ ranges from 0.6 to 14.2 and $\beta$ from -6.5 to 2.2, depending on the type of the beer being tested. The stale flavour intensity of beer is in the range of 0 to 4, exceptionally even over 4, wherein 0 denotes undamaged fresh beer without a trace of stale flavour, 1 corresponds to beer with a very mild stale flavour, 2 corresponds to beer with a mean stale flavour, 3 to beer with a strong stale flavour and 4 or more denotes beer with a very strong stale flavour.

The regression coefficient $\alpha$, for example, for bottom-fermented beers of lager type produced according to the method of Council Regulation (ES) No. 1014/2008, CHZO CZECH BEER, L276 / 27-28 ranges from 1.4 to 1.6; for wheat beers, the regression coefficient $\alpha$ is in the range of 0.6 to 0.8; for lager beers with a fermentation difference of less than 1% prepared by HGB technology it is in the range of 0.76 to 1.5; for top-fermented beers of "ale" type in the range of 0.6 to 1.35; for non-alcoholic beers produced by breaking fermentation in the range of 10 to 14.2 and for the other non-alcoholic beers it is in the range of 1.4 and 1.6.

The regression coefficient $\beta$, for example, for bottom-fermented beers of lager type produced according to the method of Council Regulation (ES) No. 1014/2008, CHZO CZECH BEER, L276 / 27-28, ranges from -0.2 to 0,2; for wheat beers, the regression coefficient $\beta$ is in the range of 1,1 to 1,4; for lager beers with a fermentation difference of less than 1% prepared by HGB technology in the range of 0.8 to 1.53; for top-fermented beers of "ale" type in the range of 1 to 2,1; for non-alcoholic beers produced by breaking fermentation in the range of -6.5 to -5.6 and for the other non-alcoholic beers it is in the range of -0.2 to 0.2.

**Description of drawings**

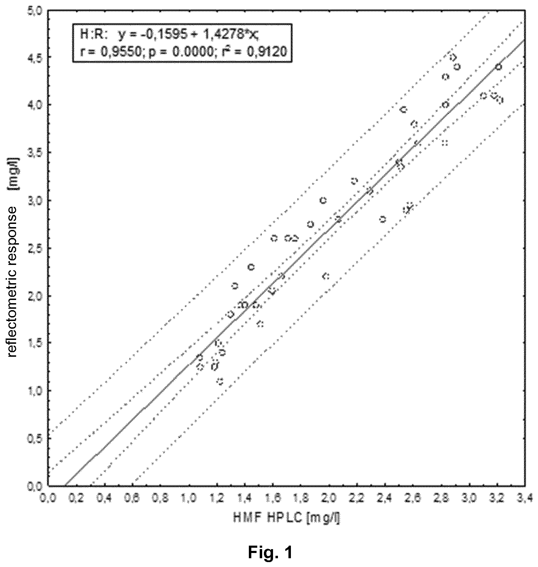

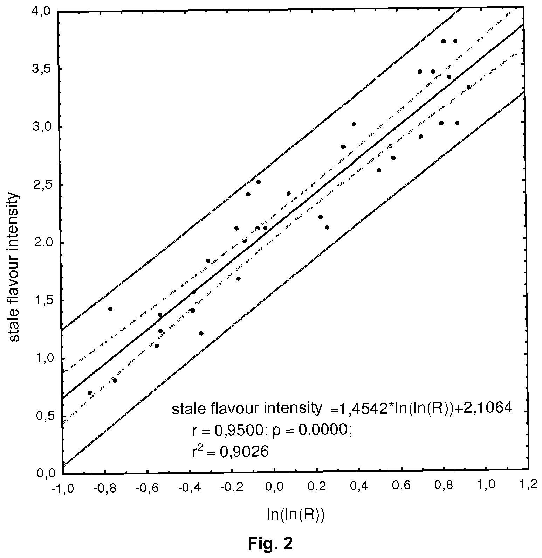

In the accompanying drawings, Fig. 1 shows the dependence of the reflectometric response on the concentration of 5-(hydroxymethyl)furan-2-carbaldehyde determined by liquid chromatography (HPLC) with spectrophotometric detection in UV spectrum, and in Fig. 2 shows the dependence of the stale flavour intensity of beer determined by an expert panel on the reflectometric response.

**Examples of embodiment**

**Example 1**

Freshly dispensed lager-type bottled beer (5 different brands supplied directly from manufacturers) were stored for 0 to 6 months at a stable temperature of 0, 20 and 30 ° C to simulate various degrees of sensory deterioration. At monthly intervals, the concentration of 5-(hydroxymethyl)furan-2-carbaldehyde was determined in the samples of these beers by liquid chromatography (HPLC) with spectrometric detection in UV spectrum. The same samples were simultaneously subjected to reflectometric analysis in which test strips were immersed in a beer sample for one second and then placed into the reflectometer measuring space. After 3 minutes (standard reflectometry method analysis time is 2 minutes), the display of the reflectometer showed a response defining the apparent sum, or, more specifically, the apparent concentration of furan-2-carbaldehydes in the given beer sample in mg/l. The results of the reflectometric analysis were checked by the reflectometric analysis of a control sample containing 5-(hydroxymethyl)furan-2-carbaldehyde at a concentration of 5 mg/l.

The relation between the results obtained by both methods was evaluated by regression analysis and a linear relationship was found between the reflectometer response and the concentration of 5-(hydroxymethyl)furan-2-carbaldehyde determined by liquid chromatography (HPLC) with spectrometric detection in UV spectrum - see Figure 1, which represents the dependence of the reflectometer response on the concentration of 5- (hydroxymethyl)furan-2-carbaldehyde determined by liquid chromatography (HPLC) with spectrophotometric detection in UV spectrum. It follows that liquid chromatography (HPLC) with spectrophotometric detection in UV spectrum can be replaced by faster and more flexible reflectometry without distorting the measurement of its results or their interpretation.

**Example 2**

The sensory profile of the beers of Example 1 was evaluated by a panel of 12 professional judges. The relationship between the extent of sensory deterioration of beer expressed by the intensity of the stale flavour in beer (indicated on a scale of 0 to 4) and the apparent sum of furan-2-carbaldehydes determined by means of reflectometric analysis was log-linearized - see Figure 2, which shows the dependence of the beer stale flavour intensity on the reflectometric response. It follows that the evaluation of the sensory beer profile by the expert panel can be replaced by a more flexible reflectometric method without distorting the results or their interpretation.

**Example 3**

Six different pale beer samples were stored at 40 ° C for 6 days. Afterwards, the intensity of beer stale flavour in them was determined by the method according to the invention. Before the actual reflectometric analysis started, the reflectometer had been recalibrated by a barcode specific to a production batch of test strips used for the determination of 5-(hydroxymethyl)furan-2-carbaldehyde, and by a blank reaction strip which had been immersed in and then removed from the beer sample to be tested. Subsequently, the reaction zone of the test strip was immersed for 1 second in the tested sample and at the same time the countdown sequence of the reflectometer was triggered, the test strip was placed in the measuring space of the reflector and, at a defined time, a reflectrometric analysis response, representing the apparent sum or the apparent concentration of furan-2-carbaldehydes in the beer sample, expressed in mg/l, was read out from the display. The defined time was 3 minutes from the immersion of the test strip in the beer sample and it differs from the time set by the reflectometer manufacturer (which normally is 2 minutes), so it was necessary to initiate a separate analysis after 1 minute from the end of the countdown sequence. The quality of the individual analyses was checked by the control sample consisting of an aqueous solution of 5-(hydroxymethyl)furan-2-carbaldehyde having a concentration of 5-(hydroxymethyl)furan-2-carbaldehyde 5.3 mg/l. Since the concentration of 5-(hydroxymethyl)furan-2-carbaldehyde in the control sample obtained as a reflectometric response was not equal to the actual concentration of 5-(hydroxymethyl)furan-2-carbaldehyde therein, the values obtained from the reflectometric analysis of the beer samples were corrected according to the above equation. The corrected reflectometric response was then used according to the method described above to quantify the intensity of the stale flavour of the beer. The results of the quantification of the stale flavour intensity of the individual samples as determined by the method according to the invention compared to the methods used so far are shown in the following table.

| Sample | $R_{sample}$ [mg/l] | $R_{QC}$ [mg/l] | $Rc_{or}$ [mg/l] | Stale flavour intensity determined by the method of the invention ($\pm$ 0,5) | Stale flavour intensity determined by HPLC (conversion according to the equation in Fig. 1) | Stale flavour intensity determined by sensory evaluation |
|---|---|---|---|---|---|---|
| Lager beer made from wort with original extract content 12% by technology according to Council Regulation (ES) No. 1014/2008, CHZO CZECH BEER, L276/27-28 | 3,7 | | 2,3 | 1,8 | 2,5 | 1,6 |
| Lager beer made from wort with original extract content 12% by technology according to Council Regulation (ES) No. 1014/2008, CHZO CZECH BEER, L276/27-28 | 3,5 | | 2,1 | 1,7 | 1,3 | 1,9 |
| Non-alcoholic beer | 2,8 | | 1,4 | < 1 | 1,2 | 0,5 |
| Beer type lager made from wort with original extract content 12% by technology according to Council Regulation (ES) No. 1014/2008, CHZO CZECH BEER, L276/27-28 | 3,9 | 6,8 | 2,5 | 2,0 | 1,8 | 1,8 |
| Lager beer made from wort with original extract content 12% by technology according to Council Regulation (ES) No. 1014/2008, CHZO CZECH BEER, L276/27-28 | 3,5 | | 2,1 | 1,7 | 2,4 | 1,7 |
| Lager beer made from wort with original extract content 12% by HGB technology | 5,4 | | 4,0 | 2,6 | 3,8 | 3,0 |

**Claims**

1. Method for *in situ* objective determination of the intensity of the stale taste of beer, **characterized in that** by means of reflectometric analysis, which takes place for 3 to 5 minutes, the apparent sum of furan-2-carbaldehydes, in particular 5-(hydroxymethyl)furan-2-carbaldehyde and furan-2-carbaldehyde in a beer sample is determined, as well as in a control sample consisting of an aqueous solution of 5-(hydroxymethyl)furan-2-carbaldehyde having a concentration of 1 to 60 mg/l, whereby:

   a) if the thus determined concentration of 5-(hydroxymethyl)furan-2-carbaldehyde in the control sample is equal to its actual concentration in the control sample, the stale flavour intensity of beer is determined according to the formula:

$$\text{stale flavour intensity} = \alpha \cdot \ln(\ln(R)) + \beta$$

where R is the apparent sum of furan-2-carbaldehydes in the beer sample detected by reflectometric analysis, expressed in mg/l, and $\alpha$ and $\beta$ are regression coefficients, whereby $\alpha$ ranges from 0.6 to 14.2 and $\beta$ ranges from -6.5 to 2.2 and the stale flavour intensity of beer is in the range from 0 to 4, exceptionally even more, when 0 denotes fresh beer without stale flavour and 4 or more denotes beer with a very strong stale flavour,

or

b) if the thus determined concentration of 5-(hydroxymethyl)furan-2-carbaldehyde in the control sample is not equal to its actual concentration, the thus determined concentration of furan-2-carbaldehydes in the beer sample is corrected according to the equation:

$$R_{cor.} = R_{sample} - (R_{QC} - conc._{QC}),$$

where $R_{kor.}$ is the corrected apparent sum of furan-2-carbaldehydes in the control sample expressed in mg/l, $R_{sample}$ is the apparent sum of furan-2-carbaldehydes in the beer sample detected by reflectometric analysis expressed in mg/l, is the reflectometric response of 5-(hydroxymethyl)furan-2-carbaldehyde in the control sample detected by reflectometric analysis expressed in mg/l and conc.$_{QC}$ is the actual concentration of 5-(hydroxymethyl)furan-2-carbaldehyde in the control sample expressed in v mg/l,

and the stale flavour intensity is subsequently determined according to the formula:

$$\text{stale flavour intensity} = \alpha \cdot \ln(\ln(R_{kor})) + \beta$$

where $R_{kor}$ is the corrected apparent concentration of furan-2-carbaldehydes in a beer sample expressed in mg/l, and $\alpha$ and $\beta$ are regression coefficients, whereby $\alpha$ ranges from 0.6 to 14.2 and $\beta$ from -6.5 to 2.2, and the stale flavour intensity of beer is in the range of 0 to 4, exceptionally even more, wherein 0 denotes fresh beer without stale flavour and 4 and more denotes beer with a very strong stale flavour.

2. The method according to claim 1, **characterized in that** the regression coefficient $\alpha$ has a value between 1.4 and 1.6 for lager beers produced according to the method of the Council Regulation (ES) No. 1014/2008, CHZO CESKE PIVO, L276/27-28; a value between 0.6 and 0.8 for wheat beers; a value between 0.76 to 1.5 for lager beers with a fermentation difference of less than 1%, prepared by HGB technology; a value between 0.6 to 1.35 for top-fermented beers of "ale" type; a value between 10 and 14.2 for non-alcoholic beers produced by technology of breaking fermentation; a value between 1.4 and 1.6 for the other non-alcoholic beers, and the regression coefficient $\beta$ ranges from -0.2 to 0.2 for lager beers produced according to the method of the Council Regulation (ES) No. 1014/2008, CHZO CESKE PIVO, L276/27-28; from 1.1 to 1.4 for wheat beers; from 0.8 to 1.53 for lager beers with a fermentation difference of less than 1%, prepared by HGB technology; from 1 to 2.1 for top-fermented beers of "ale" type; from -6.5 to -5.6 for non-alcoholic beers produced by the technology of breaking fermentation; from -0.2 to 0.2 for the other non-alcoholic beers.

3. The method according to claim 1, **characterized in that** the control sample contains 5-(hydroxymethyl)furan-2-carbaldehyde at a concentration of 1 to 20 mg/l.

4. The method according to claim 1 or 3, **characterized in that** the control sample contains 5-(hydroxymethyl)furan-2-carbaldehyde at a concentration of 1.5 to 7 mg/l.

5. The method according to claim 1, **characterized in that** the reflectometric analysis takes place for 3 minutes.

6. The method according to claim 1, **characterized in that** the reflectometric analysis is performed on a mobile reflectometer.

**Fig. 1**

stale flavour intensity $=1{,}4542*\ln(\ln(R))+2{,}1064$

$r = 0{,}9500$; $p = 0.0000$;

$r^2 = 0{,}9026$

**Fig. 2**